# EUROPEAN PATENT SPECIFICATION

(11) **EP 1 005 530 B1**
(45) Date of publication and mention of the grant of the patent: **15.03.2006**
(21) Application number: 98918955.0
(22) Date of filing: 05.04.1998
(51) Int. Cl.: C12N 1/21, C12N 15/74, C12N 15/52, C12P 7/06, C12N 15/70

(54) **SINGLE ZYMOMONAS MOBILIS STRAIN FOR XYLOSE AND ARABINOSE FERMENTATION**
EINZELNER ZYMOMONAS MOBILIS STAMM ZUR FERMENTATION VON XYLOSE UND ARABINOSE
SOUCHE UNIQUE DE ZYMOMONAS MOBILIS POUR FERMENTATION DE XYLOSE ET D'ARABINOSE

(30) Priority: 06.05.1997 US 851767
(43) Date of publication of application: 07.06.2000
(73) Proprietor: Midwest Research Institute, Kansas City MO 64110 (US)
(72) Inventor: ZHANG, Min, Lakewood, CO 80228 (US); CHOU, Yat-Chen, Wheat Ridge, CO 80033 (US); PICATAGGIO, Stephen, K., Landerberg, PA 19350 (US); FINKELSTEIN, Mark, Fort Collins, CO 80526 (US)
(74) Representative: Wagner, Karl H.
(86) International application number: PCT/US1998/009171
(87) International publication number: WO 1998/050524

(56) References cited:
- EP-A- 0 737 742
- US-A- 5 712 133
- BOTHAST R J ET AL: "FERMENTATION OF L-ARABINOSE, D-XYLOSE AND D-GLUCOSE BY ETHANOLOGENIC RECOMBINANT KLEBSIELLA OXYTOCA STRAIN P2" BIOTECHNOLOGY LETTERS, KEW, SURREY, GB, vol. 16, no. 4, 1 April 1994 (1994-04-01), pages 401-406, XP000651597 ISSN: 0141-5492
- DEANDA K. et al., "Development of an Arabinose-Fermenting Zymomonas Mobilis Strain by Metabolic Pathway Engineering", APPLIED AND ENVIRONMENTAL MICROBIOLOGY, December 1996, Vol. 62, No. 12, pages 4465-4470, XP002912122
- ZHANG M. et al., "Metabolic Engineering of a Pentose Metabolism Pathway in Ethanologenic Zymomonas Mobilis", SCIENCE, 3 January 1995, Vol. 267, pages 240-243, XP002912123

## Description

### Field of the Invention:

This invention relates to a recombinant *Zymomonas mobilis* biocatalyst capable of converting xylose and arabinose along with glucose to ethanol.

This is a new, single *Zymomonas mobilis* biocatalyst capable of converting both xylose and arabinose along with glucose to ethanol. Seven genes (xylose isomerase, xylulokinase, L-arabinose isomerase, L-ribulokinase-5-P 4-epimerase, transketolase, and transaldolase) which encode the enzymes necessary for converting xylose and arabinose to common intermediates of *Zymomonas'* central glycolic pathway were simultaneously introduced into *Zymomonas* under the control of strong promoters that direct their expression, even in the presence of glucose. The newly engineered strain can grow on and ferment either xylose, arabinose, or glucose as sole carbon sources to ethanol and ferments a mixture of 1% glucose, 2% xylose and 2% arabinose to ethanol at about 90% of the theoretical yield at 30°C without pH control.

### Background Art:

Cellulosic biomass is a favorable feedstock for fuel ethanol production because it is both readily available and less expensive than either corn or sugarcane. However, substantial hurdles must be overcome before a typical cellulosic feedstock can be utilized effectively as a substrate for the fermentative production of ethanol. The typical feedstock is comprised of approximately 35%-45% cellulose, 30-40% hemicellulose, 15% lignin and 10% of other components. The cellulose fraction is comprised of polymers of the hexose sugar, glucose. The hemicellulose fraction is comprised mostly of pentose sugars, and substantially of xylose.

Whereas microorganisms are known that can efficiently ferment the glucose component in cellulose, conversion of the xylose in the hemicellulose fraction to ethanol has been difficult and this remains to be one of the economical bottlenecks in the biomass to ethanol conversion scheme. The rapid and efficient utilization of the xylose component in cellulosic biomass is desirable in the development of a commercial process.

*Zymomonas mobilis is* a bacterium that has been utilized as a natural fermentative agent in the production of alcoholic beverages, such as pulque and palm wines produced from plant saps. Comparative performance trials have suggested that *Zymomonas* may become an important industrial ethanol-producing microorganism because of its 5-10% higher yield and up to 5-fold higher productivity compared to traditional yeast fermentations. Because of its potential value, several processes based on the use of Zymomonas for production of industrial ethanol from glucose-based feedstocks have been disclosed in U.S. Pat. Nos. 4,731,329, 4,812,410, 4,816,399, and 4,876,196.

While *Zymomonas* may become an important fuel ethanol-producing microorganism from glucose-based feedstocks, its substrate utilization range is restricted to fermentation of glucose, sucrose and fructose and, as such, it is not naturally suited for fermentation of the xylose component in cellulosic feedstocks. Zymomonas contains the Enter-Douderoff pathway that allows it to ferment glucose very efficiently to ethanol as the sole fermentation product. However, *Zymomonas is* naturally unable to ferment the xylose in cellulosic biomass because it lacks the essential pentose metabolism pathways. Thus, an opportunity exists to genetically engineer this organism for the fermentation of xylose to ethanol.

Genetic engineering attempts have been made to enhance ethanol production by fermentation by transferring genes from one species to another. For example, see U.S. Pat. Nos. 5,000,000 and 5,028,539. Gene cloning and expression of various enzymes including enzymes for creating a new metabolic pathway are also known. For example see U.S. Pat. Nos. 5,272,073, 5,041,378, 5,168,056 and 5,226,475. However, none of these discoveries has successfully broadened the fermentable substrate range of a microorganism which could not previously ferment xylose to ethanol.

Previous attempts to introduce a xylose catabolic pathway from other Xanthomonas or Klebsiella into Zymomonas have been unsuccessful and the recombinant strains were incapable of growth on xylose as the sole carbon source (Feldmann et al., 1992, Appl. Microbiol. BiotechnoL 38:354-361; Liu et aL, 1988. J. Biotechnol. 7:61-77)

The wild-type Z. *mobilis* ferments only glucose, sucrose and fructose and is not able to utilize pentoses, such as D-xylose and L-arabinose. Enzymatic analysis indicated that Z. mobilis lacks functional pentose metabolic pathways necessary to ferment D-xylose and L-arabinose. We have developed the xylose-fermenting *Z. mobilis* by introduction and expression of four genes encoding D-xylose-assimilating enzymes, xylose isomerase and xylulokinase as well as pentose-phosphate pathway enzymes, transaldolase and transketolase (Picataggio et al., U.S. 5,514,583). By introducing the genes encoding xylose isomerase and xylulokinase, xylose cam be converted to xylulose 5 P. Then, by introducing two more genes encoding enzymes in the pentose phosphate pathway, comprising transaldolase and transketolase, xylulose-5-P can be further converted to the key intermediates that couple pentose metabolism to the glycolytic Entner-Douderoff pathway and consequently, to ethanol production. Independently, we have also developed the arabinose-fermenting Z. *mobilis* by introduction of five genes encoding L-arabinose-assimilating enzymes, L-arabinose isomerase, L-ribulokinase and L-ribulose-5-phosphate 4-epimerase as well as the pentose-phosphate pathway enzymes, transaldolase and transketolase (U.S. 08/421,996). By introducing the genes encoding L-arabinose isomerase, L-ribulokinase and L-ribulose-5-phosphate-4-epimerase, arabinose can be converted to xylulose-5-P. Similarly, by introducing the genes encoding enzymes in the pentose phosphate pathway, comprising transaldolase and transketolase, xylulose-5-P can be further converted to the key intermediates that couple pentose metabolism to the glycolytic Entner-Douderoff pathway and consequently, to ethanol production.

EP-A-0 737 742 relates to microorganisms which normally do not ferment pentose sugar and which are genetically altered to ferment pentose sugar to produce ethanol, and fermentation processes utilizing the same. Examples include *Zymomonas mobilis* which has been transformed with combinations of *E. coli* genes for xylose isomerase, xylulokinase, transaldolase, transketolase, L-arabinose isomerase, L-ribulokinase, and L-ribulose 5-phosphate 4-epimerase. Expression of the added genes are under the control of *Zymomonas mobilis* promoters. These microorganisms are useful for fermenting pentoses and glucose, produced by hydrolysis of hemicellulose and cellulose, to produce ethanol.

BOTHAST R. J: et al.: "FERMENTATION OF L-ARABINOSE, D-XYLOSE AND D-GLUCOSE BY ETHANOLOGENIC RECOMBINANT *KLEBSIELLA OXYTOCA* STRAIN P2" BIOTECHNOLOGY LETTERS, KEW, SURREY, GB, vol. 16, no. 4, 1 April 1994 (1994-04-01), pages 401-406, XP000651597, ISSN: 0141-5492, discloses an evaluation of recombinant *Klebsiella oxytoca* strain P2 carrying genes for pyruvate decarboxylase and alcohol dehydrogenase from *Zymomonas mobilis* for its ability to ferment arabinose, xylose and glucose alone and in mixtures in pH-controlled batch fermentations. This organism produced 0.34-0.43 g ethanol/g sugar at pH 6.0 and 30°C on 8% sugar substrate and demonstrated a preference for glucose. Sugar utilization was glucose > arabinose > xylose and ethanol production was xylose > glucose > arabinose.

### Disclosure of the Invention:

The present invention provides a single *Zymomonas mobilis* biocatalyst capable of converting both xylose and arabinose along with glucose to ethanol. Seven genes (xylose isomerase, xylulokinase, L-arabinose isomerase, L-ribulokinase, L-ribulose-5-P 4-epimerase, transketolase, and transaldolase) which encode the enzymes necessary for converting xylose and arabinose to common intermediates of *Zymomonas'* central glycolytic pathway were simultaneously introduced into *Zymomonas* under the control of strong promotors that direct their expression, even in the presence of glucose. The newly engineered strain can grow on and ferment either xylose, arabinose, or glucose as sole carbon sources to ethanol and ferments a mixture of 1% glucose, 2% xylose and 2% arabinose to ethanol at about 90% of the theoretical yield at 30°C without pH control.

A further aspect of the present invention is to enable the Z. *mobilis* to ferment both xylose and arabinose, by introducing and expressing the genes for both xylose-assimilating enzymes, xylose isomerase and xylulokinase, and L-arabinose-assimilating enzymes, L-arabinose isomerase, L-ribulokinase and L-ribulose-5-phosphate 4-epimerase, as well as the pentose-phosphate pathway enzymes, transaldolase and transketolase into Z. *mobilis.* The cloned genes may be provided on any number of vectors but preferably are contained on a single plasmid vector. More preferably, the genes are integrated into the host genome.

Another aspect of the present invention is cultures of microorganisms with the above-described abilities. The cultures may be biologically pure or be mixed with other strains of different organisms to aid in the metabolism of the substrates or a mixture of substrates into ethanol.

Yet another aspect of the invention is a process for producing a newly engineered strain that can grow on and ferment either xylose, arabinose or glucose as sole carbon sources to ethanol.

An additional aspect of the invention is to provide a new *Zymomonas* strain that will be useful for those lignocellulosic feedstocks containing significant quantities of both the pentose sugars (such as switch grass, wheat straw, corn cobs, corn fiber and spent grains).

### Brief Description of the Drawings:

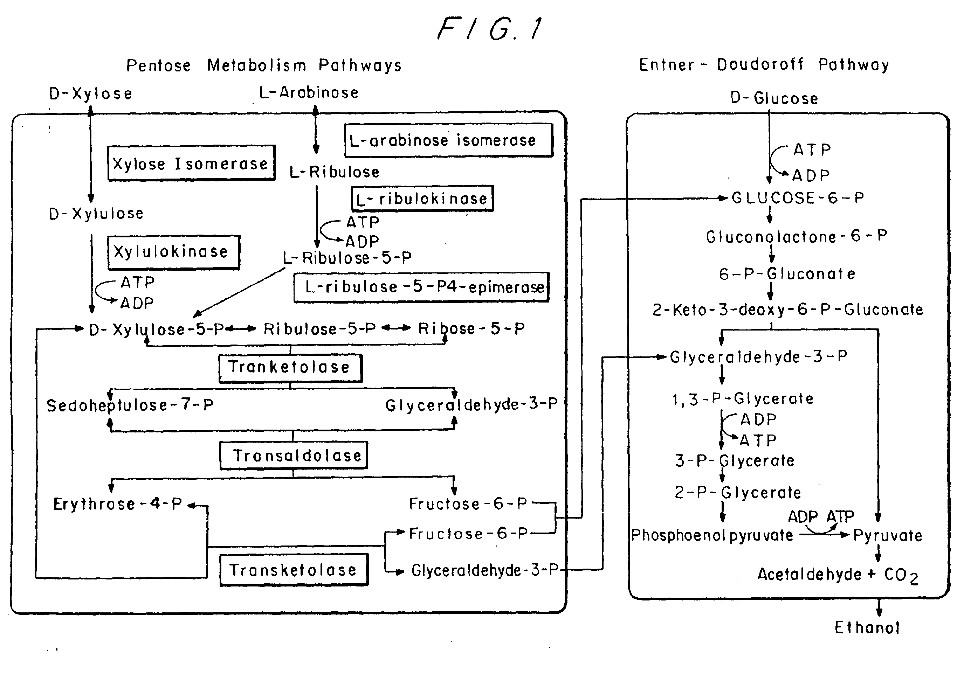

Figure 1 shows a schematic of a process of enabling Z. *mobilis* to ferment both xylose and arabinose by introducing and expressing the genes from both xylose-assimilating enzymes, xylose isomerase and xylulokinase, and L-arabinose-assimilating enzymes, L-arabinose isomerase, L-ribulokinase and L-ribulose-5-phosphate 4-epimerase, as well as the pentose-phosphate pathway enzymes, transaldolase and transketolase into Z. mobilis.
Figure 2 depicts the plasmid maps pZB301, pZB401, pZB402, and pZB403.
Figure 3 shows ethanol yields using a control Zymomonas mobilis and the present recombinant strain when grown on certain sugars or a mixture of certain sugars as the carbon source.

### Description of Preferred Embodiments:

The invention is the preparation of plasmids of all seven genes, xylose isomerase, xylulokinase, L-arabinose isomerase, L-ribulokinase, L-ribulose-5-phosphate 4-epimerase, transaldolase and transketolase under control of strong, constitutive Z. mobilis promoters that allow high expression of all the genes and transformed Z. *mobilis.* Specifically, xylose isomerase and xylulokinase are controlled under the Z. *mobilis* glyceraldehyde-3-phosphate dehydrogenase (GAP) promoter in a P_{gap}-xylAxyIB operon; L-arabinose isomerase, L-ribulokinase and L-ribulose-5-phosphate 4-epimerase are also controlled under the Z. mobilis GAP promoter in a P_{gap}-xylAxylB operon; transaldolase and transketolase are controlled under the Z. *mobilis* enolase (ENO) promoter in a Pₑₙₒ-tal/tkt operon.

### Example I

### Selection of Appropriate Z. mobilis Hosts for Transformation and Expression of Arabinose and Xylose Utilization Genes

While the wild-type strain of Z. mobilis 39676 transformed with pZB4L carrying the genes for four xylose metabolism enzymes acquired Xyl⁺ phenotype, transformation of pZB206 into the wild-type Z. mobilis 39676 did not provide the ability to grow on L-arabinose containing media. It was apparent that mutation event (s) required for conferring Ara+ in addition of expressing ara genes in Z. *mobilis* might have occurred on the chromosomal DNA of the arabinose-fermenting Z. *mobilis* 39676(pZB206) strain when constructed. Therefore, efforts were made to cure pZB206 from the strain 39676 (pZB206) to obtain an appropriate host strain by culturing the strain in a non-selective medium (i.e. without tetracycline (Tc)) at 37°C for about 20 generations. The cells lost from the plasmid were confirmed by testing its Tc sensitivity and Xyl phenotype through replica plating as well as by mini-screening of the plasmid. The pZB206 cured strain was designed Z. *mobilis* 206C. Transformation of this strain individually with pZB206 (arabinose plasmid) (Ser. No. 08/421,996), pZB4 (xylose plasmid) (Picataggio et al., U.S. 5,514,583), and pZB4L (xylose plasmid) separately conferred Ara+ and Xyl⁺ phenotypes to the corresponding transformants. These results indicated the Z. *mobilis* 206C carried mutation(s) contributing to Ara+ phenotypes. Therefore, Z. *mobilis* 206C was chosen to be the host for expressing both arabinose and xylose utilization genes.

### Example II

### Expression of Xylose and Arabinose Utilization Genes in Z. mobilis 206C

Plasmids were constructed containing all seven genes, xylose isomerase, xylulokinase, L-arabinose isomerase, L-ribulokinase, L-ribulose-5-phosphate 4-epimerase, transaldolase and transketolase under control of strong, constitutive Z. *mobilis* promotors that allow high expression of all the genes and transformed Z. *mobilis.* Specifically, xylose isomerase and xylulokinase are controlled under the Z. *mobilis* glyceraldehyde-3-phosphate dehydrogenase (GAP) promotor in a P_{gap}-xylAxylB operon; L-arabinose isomerase, L-ribulokinase and L-ribulose-3-phosphate 4-epimerase are also controlled under the Z. *mobilis* GAP promotor in a P_{gap}-xylAxylB operon; transaldolase and transketolase are controlled under the Z. *mobilis* enolase (ENO) promotor in a Pₑₙₒ-tal/tkt operon.

### Example III

### Development Of A Single Strain With Both Xylose And Arabinose-Fermentating Capability

To develop a single strain with both xylose and arabinose-fermentating capability a single plasmid was constructed containing xylose and arabinose utilization genes which includes xylose isomerase, xylulokinase, L-arabinose isomerase, L-ribulokinase, L-ribulose-5-phosphate-4-epimerase, transaldolase, and transketolase. The xylose plasmid pZB4 was partially digested with NotI followed by ligation with a gel purified 4.4-kb NotI fragment containing P_{gap}-araBAD operon from pZB206. The ligation mixture was used to electroporate E. *coli* B/r araA - and cells were plated on MacConkey plates containing L-arabinose and Tc.

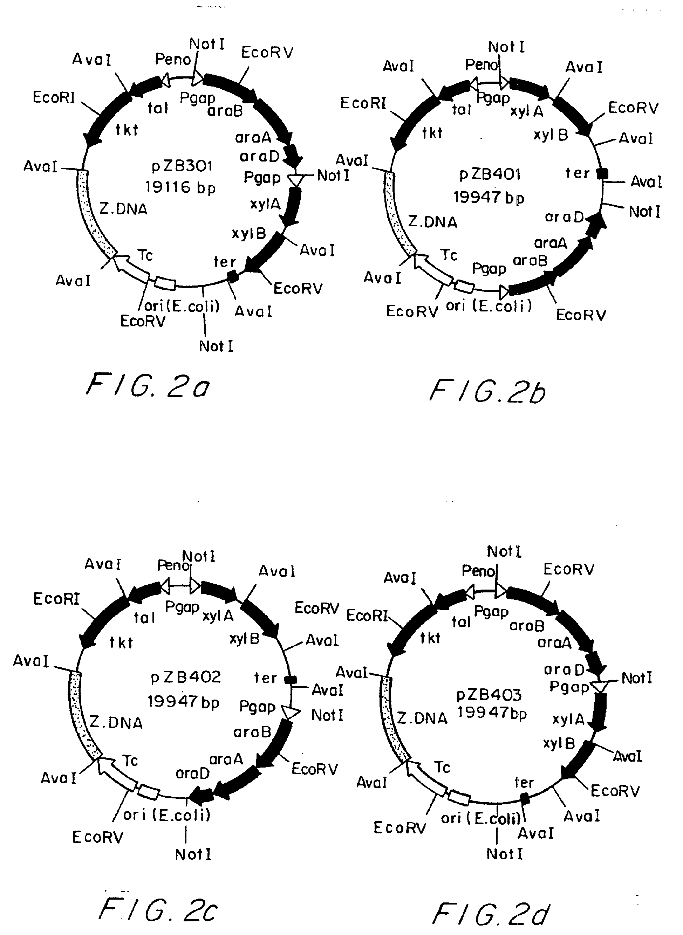

The Ara⁺Tc^{r} transformants were examined for the presence of the correct P_{gap-}araBAD operon insert in pZB4. DNA analysis of transformants indicated that NotI insert containing P_{gap}-araBAD operon was inserted clockwise in the NotI site upstream to P_{gap-}xyl/AxylB operon. The resulting plasmid, designated as pZB301 (Fig. 2), was then used to electroporate Z. *mobilis*. Z. mobilis 206C transformed with pZB301 was able to grow on media containing either L-arabinose or D-xylose as sole carbon source in the presence of Tc.

### Example IV

### Preparation of a Single Plasmid containing Xylose and Arabinose Utilization Genes Based on Another Xylose Plasmid pZBL

Efforts were made to construct a single plasmid containing xylose and arabinose utilization genes based on another xylose plasmid pZB4L. Similarly, pZB4L was digested with NOTI followed by ligation with a gel purified 4.4-kb NotI fragment containing P_{gap-}araBAD operon from PZB206. *E. Coli* HB101 (instead of B/r ara A-) was electroporated with the ligation mixture followed by selection on MacConkey+ara+Tc plates. The Ara⁺Tc^{r} transformants were examined for the presence of the correct P_{gap}-araBAD operon insert in pZB4L. DNA analysis indicated three types of orientations of the insert. These plasmids were designated as pZB401, pZB402, and pZB403 (Fig. 2), and were then used to electroporate Z. *mobilis* 206C. Z. *mobilis* 206C transformed with pZB401, pZB402 or pZB403 and were able to grow on media containing either L-arabinose or D-xylose as a sole carbon source in the presence of To.

Enzymatic analysis confirmed that all seven enzymes are highly expressed as compared to the control strain containing vector pZB 186 (Table 1).

**Table 1. Summary of Enzymatic Assays**

| Strains¹ | Specific Enzyme Activity (U/Mg protein) | | | | | | |
|---|---|---|---|---|---|---|---|
| | X1 | XK | L-AI | L-RK | L-Repi | TKT | TAL |
| 206C(pZB186) | 0.03 | 0.08 | nd² | 0.58 | 0.01 | 0.01 | 0.07 |
| 206C(pZB301) | 0.29 | 3.30 | 3.25 | 1.34 | 0.22 | 0.55 | 2.04 |
| 206C(pZB401) | 0.25 | 0.82 | 2.22 | 1.56 | 0.29 | 0.57 | 2.25 |

| | | | | | | | |
|---|---|---|---|---|---|---|---|
| ¹Cells were collected at OD₆₀₀=1.2. | | | | | | | |
| ²nd=not detected | | | | | | | |

### Fermentation Performance of the Combined Xylose and Arabinose-Fermenting Z. mobilis strains

To investigate the fermentation performance of these newly constructed combined xylose and arabinose-fermenting Z. *mobilis* strains, fermentations were performed statically at 30°C without pH control in bottles with 80 ml RM+Tc containing mixtures of 2% arabinose +2% xylose or 1% glucose +2% arabinose +2% xylose for strains of 206C(pZB301), 206C(pZB401), 206C(pZB402) and 206C (pZB403).

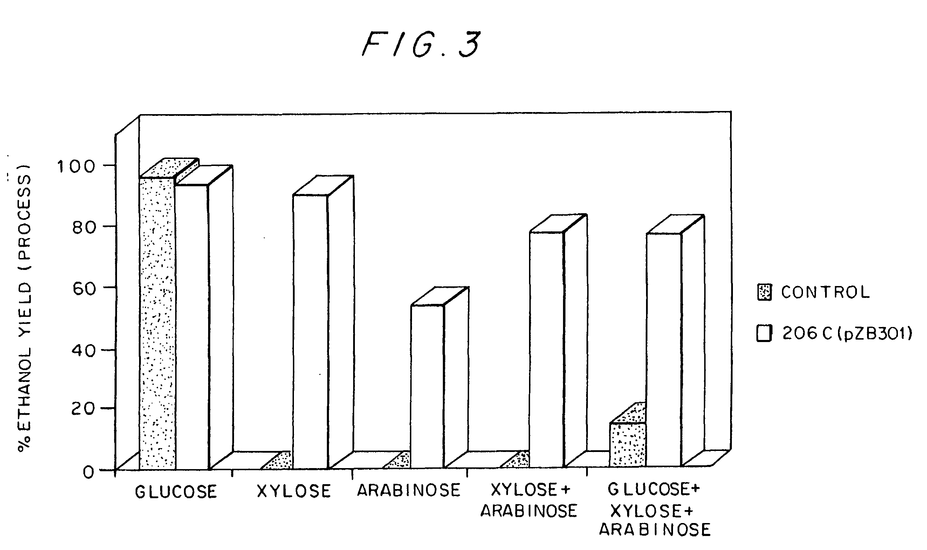

In the absence of glucose, all four strains fermented a mixture of 2% xylose and 2% arabinose at 63-66% of ethanol yields (process) in about 48 hours. The ethanol yields based on consumed sugars ranged from 0.45 to 0.48 g/g. The low process yields resulted from residual arabinose, apparently due to a decrease in the pH during fermentation without pH control. Although it is not clear whether both arabinose and xylose were transported into the cell by the same sugar transport system it appears that the presence of the arabinose does not inhibit xylose utilization and vice versa. The sugars are simultaneously utilized. However, the rate of the utilization is much higher in xylose as compared with arabinose. A representative fermentation profile is shown in Figure 3.

In the presence of 1% glucose, all four strains fermented a mixture of 2% xylose and 2% arabinose at 72-75% of ethanol yields (process) in about 48 hours. The ethanol yields based on consumed sugars ranged from 0.44 to 0.48 g/g. Glucose is preferentially utilized at a faster rate than xylose and arabinose. The growth started earlier and reached about 1.6-2 fold higher OD for all strains than in the absence of glucose. Apparently, addition of the glucose facilitated pentose utilization and resulted in higher process yields.

## Claims

1. A microorganism of the genus Zymomonas containing exogenous genes encoding xylose isomerase, xylulokinase, L-arabinose isomerase, L-ribulokinase, L-ribulose-5-phosphate 4-epimerase, transaldolase and transketolase and further comprising at least one promoter recognized by *Zymomonas* which regulates the expression of at least one of said genes, wherein said microorganism is capable of growing on arabinose and xylose as carbon sources and fermenting said arabinose and xylose to ethanol in about 75% theoretical yield and, wherein said microorganism without said genes is incapable of growing on or fermenting said arabinose and xylose to ethanol.

2. A microorganism according to claim 1, wherein the genes encoding xylose isomerase, xylulokinase, transaldolase and transketolase were obtained from bacteria selected from the group consisting of Xanthomonas, Klebsiella, E. coli, Rhodobacter, Flavobacterium, Acetobacter, Gluconobacter, Rhizobium, Agrobacterium, Salmonella and Pseudomonads.

3. A microorganism according to claim 1, wherein the genes are integrated into the host genome.

4. A microorganism according to claim 1, wherein said genes are contained on a vector.

5. A microorganism according to claim 4, wherein said xylose isomerase and xylulokinase are expressed under the control of a glyceraldehyde-3-phosphate based dehydrogenase promoter in a P_{gap}-xylA xylB operon promoter recognized by *Zymomonas;* L-arabinose isomerase, L-ribulokinase and L-ribulosc-5-phosphate 4-epimerase are also expressed under the control of glyceraldehyde-3-phosphate dehydrogenase promoter in a P_{gap}-xylA xylB operon recognized by Zymomonas; and transaldolase and transketolase are expressed under the control of a enolase promoter in a Peₙₒ-tal/tkt operon recognized by *Zymomonas.*

6. A vector comprising genes encoding xylose isomerase, xylulokinase, L-arabinose isomerase, L-ribulokinase, L-ribulose-5-phosphate 4-epimerase, transaldolase and transketolase and at least one promoter recognized by *Zymomonas* which regulates expression of said genes.

7. A vector according to claim 6, wherein the enzymes xylose isomerase and xylulokinase are regulated by a glyceraldehyde-3-phosphate dehydrogenase promotor in a P_{gap}-xylA xylB operon recognized by *Zymomonas*.

8. A vector according to claim 6, wherein the genes encoding L-arabinose isomerase, L-ribulokinase and L-ribulose-5-phosphate 4-epimerase are regulated by a glyceraldehyde-3-phosphate dehydrogenase promoter in a P_{gap}-xylA xylB operon recognized by Zymomonas.

9. A vector according to claim 6, wherein the genes encoding transaldolase and transketolase are regulated by an enolase promoter in a Pₑₙₒ-tal/tkt operon recognized by *Zymomonas*.

10. A microorganism according to claim 2, wherein the genes encoding xylose isomerase, xylulokinase, transaldolase and transketolase are obtained from E. *coli.*

11. Use of the microorganism of the genus Zymomonas of claim 1 to ferment xylose and/or arabinose, along with glucose, produced from a cellulosic biomass feedstock, to ethanol.

## Patentansprüche

1. Ein Mikroorganismus der Gattung *Zymomonas,* der exogene für Xylose-Isomerase, Xylulokinase, L-Arabinose-Isomerase, L-Ribulokinase, L-Ribulose-5-Phosphat 4-Epimerase, Transaldolase und Transketolase kodierende Gene enthält und ferner mindestens einen Promotor umfasst, der von *Zymomonas* erkannt wird, welcher die Expression mindestens eines der Gene reguliert, wobei der Mikroorganismus fähig ist auf Arabinose und Xylose als Kohlenstoffquelle zu wachsen und Arabinose und Xylose mit ungefähr 75% der theoretischen Ausbeute zu Ethanol zu fermentieren und, wobei der Mikroorganismus ohne diese Gene unfähig ist auf Arabinose und Xylose zu wachsen oder diese zu Ethanol zu fermentieren.

2. Ein Mikroorganismus gemäß Anspruch 1, wobei die für Xylose-Isomerase, Xylulokinase, Transaldolase und Transketolase kodierenden Gene aus Bakterien gewonnen wurden, die aus der aus *Xanthomonas, Klebsiella, E. coli, Rhodobacter, Flavobacterium, Acetobacter, Gluconcobacter, Rhizobium, Agrobacterium, Salmonella* und *Pseudomonaden* bestehenden Gruppe ausgewählt wurden.

3. Ein Mikroorganismus gemäß Anspruch 1, wobei die Gene in ein Gastgenom integriert sind.

4. Ein Mikroorganismus gemäß Anspruch 1, wobei die Gene auf einem Vektor enthalten sind.

5. Ein Mikroorganismus gemäß Anspruch 4, wobei Xylose-Isomerase und Xylulokinase unter der Steuerung eines auf Glycerinalhdeyd-3-Phoshat basierenden Dehydrogenase-Promotors in einem von *Zymomonas* erkannten P_{gap}-xylA xylB Operon-Promotor exprimiert werden; L-Arabinose-Isomerase, L-Ribulokinase, L-Ribulose-5-Phosphat 4-Epimerase werden ebenfalls unter der Steuerung des Glycerinaldehyd-3-phoshat-Dehydrogenase-Promotors in einem von *Zymomonas* erkannten P_{gap}-xylA xylB Operon exprimiert; und Transaldolase und Transketolase werden unter der Steuerung eines Enolase-Promotors in einem von *Zymomonas* erkannten Pₑₙₒ₋tal/tkt-Operon exprimiert.

6. Ein Vektor, der für Xylose-Isomerase, Xylulokinase, L-Arabinose-Isomerase, L-Ribulokinase, L-Ribulose-5-Phosphat 4-Epimerase, Transaldolase und Transketolase kodierende Gene und mindestens einen von *Zymomonas* erkannten Promotor umfasst, der die Expression dieser Gene reguliert.

7. Ein Vektor gemäß Anspruch 6, wobei die Enzyme Xylose Isomerase und Xylulokinase durch einen Glycerinaldehyd-3-phoshat-Dehydrogenase-Promotor in einem von *Zymomonas* erkannten P_{gap}-xylA xylB Operon reguliert werden

8. Ein Vektor gemäß Anspruch 6, wobei die für L-Arabinose Isomerase, L-Ribulokinase und L-Ribulose-5-Phosphat 4-Epimerase kodierenden Gene durch einen Glycerinaldehyd-3-phoshat-Dehydrogenase-Promotor in einem von *Zymomonas* erkannten P_{gap}-xylA xylB Operon reguliert werden.

9. Ein Vektor gemäß Anspruch 6, wobei die für Transaldolase und Transketolase kodierenden Gene durch einen Enolase-Promotor in einem von *Zymomonas* erkannten Pₑₙₒ-tal/tkt-Operon reguliert werden.

10. Ein Mikroorganismus gemäß Anspruch 2, wobei die für Xylose-Isomerase, Xylulokinase, Transaldolase und Transketolase kodierenden Gene aus *E. coli* gewonnen werden.

11. Verwendung des Mikroorganismus der Gattung *Zymomonas* gemäß Anspruch 1, um Xylose und/oder Arabinose, zusammen mit Glukose, die aus einem zellulosischen Biomasse-Rohmaterial produziert wird, zu Ethanol zu fermentieren.

## Revendications

1. Microorganisme du genre Zymomonas contenant des gènes exogènes codant pour la xylose isomérase, la xylulokinase, la L-arabinose isomérase, la L-ribulokinase, la L-ribulose-5-phosphate 4-épimérase, la transaldolase et la transketolase et comprenant en outre au moins un promoteur reconnu par Zymomonas qui régule l'expression d'au moins un desdits gènes, dans lequel le microorganisme peut croître sur l'arabinose et le xylose en tant que sources de carbone et fermenter l'arabinose et le xylose en éthanol avec un rendement théorique d'environ 75 % et dans lequel le microorganisme sans lesdits gènes est incapable de croître sur ou de fermenter en éthanol l'arabinose et le xylose.

2. Microorganisme selon la revendication 1, dans lequel les gènes codant pour la xylose isomérase, la xylulokinase, la transaldolase et la transketolase sont obtenus à partir de bactéries choisies dans le groupe constitué de Xanthomonas, Klebsiella, E. coli, Rhodobacter, Flavobacterium, Acetobacter, Gluconobacter, Rhizobium, Agrobacterium, Salmonelle et Pseudomonas.

3. Microorganisme selon la revendication 1, dans lequel les gènes sont intégrés dans le génome de l'hôte.

4. Microorganisme selon la revendication 1, dans lequel les gènes sont contenus dans un vecteur.

5. Microorganisme selon la revendication 4, dans lequel la xylose isomérase et la xylulokinase sont exprimées sous le contrôle d'un promoteur déshydrogénase à base de glycéraldéhyde-3-phosphate dans un promoteur opéron P_{gap}-xylA xylB reconnu par Zymomonas ; la L-arabinose isomérase, la L-ribulokinase et la L-ribulose-5-phosphate 4-épimérase sont également exprimées sous le contrôle du promoteur glycéraldéhyde-3-phosphate déshydrogénase dans un opéron P_{gap-}xylA xylB reconnu par Zymomonas ; et la transaldolase et la transketolase sont exprimées sous le contrôle d'un promoteur enolase dans un opéron Pₑₙₒ-tal/tkt reconnu par Zymomonas.

6. Vecteur comprenant des gènes codant pour la xylose isomérase, la xylulokinase, la L-arabinose isomérase, la L-ribulokinase, la L-ribulose-5-phosphate 4-épimérase, la transaldolase et la transketolase et au moins un promoteur reconnu par Zymomonas qui régule l'expression de ces gènes.

7. Vecteur selon la revendication 6, dans lequel les enzymes xylose isomérase et xylulokinase sont régulées par un promoteur glyeéraldéhyde-3-phosphate déshydrogénase dans un opéron P_{gap}-xylA xylB reconnu par Zymomonas.

8. Vecteur selon la revendication 6, dans lequel les gènes codant pour la L-arabinose isomérase, la L-ribulokinase et L-ribulose-5-phosphate 4-épimérase sont régulés par un promoteur glycéraldéhyde-3-phosphate déshydrogénase dans un opéron P_{gap-}xylA xylB reconnu par Zymomonas.

9. Vecteur selon la revendication 6, dans lequel les gènes codant la transaldolase et la transketolase sont régulés par un promoteur enolase dans un opéron Pₑₙₒ-tal/tkt reconnu par Zymomonas.

10. Microorganisme selon la revendication 2, dans lequel les gènes codant pour la xylose isomérase, la xylulokinase, la transaldolase et la transketolase sont obtenus à partir de E. coli.

11. Utilisation du microorganisme du genre Zymomonas selon la revendication 1 pour fermenter le xylose et/ou l'arabinose, en combinaison avec du glucose, produit à partir d'un substrat de biomasse cellulosique, en éthanol.
